# FASCICULE DE BREVET EUROPEEN

(11) **EP 2 956 438 B1**
(45) Date de publication et mention de la délivrance du brevet: **22.03.2017**
(21) Numéro de dépôt: 14710600.9
(22) Date de dépôt: 14.02.2014
(51) Int. Cl.: C07D 211/22, C07B 35/02, C07D 211/52

(54) **PROCEDE DE SYNTHESE DE 4-PIPERIDIN-4-YL-BENZENE-1,3-DIOL ET DE SES SELS ET COMPOSE 4-(2,4-DIHYDROXY-PHENYL)-4-HYDROXY-PIPERIDINE-1-CARBOXYLATE DE TERT-BUTYLE**
VERFAHREN ZUR SYNTHESE VON 4-PIPERIDIN-4-YL-BENZOL-1,3-DIOL UND SALZEN DAVON SOWIE NEUARTIGE VERBINDUNG TERT-BUTYL-4-(2,4-DIHYDROXY-PHENYL)-4-HYDROXY-PIPERIDIN-1-CARBOXYLAT
METHOD FOR SYNTHESISING 4-PIPERIDIN-4-YL-BENZENE-1,3-DIOL AND THE SALTS OF SAME AND COMPOUND TERT-BUTYL 4-(2,4-DIHYDROXY-PHENYL)-4-HYDROXY-PIPERIDINE-1-CARBOXYLATE

(30) Priorité: 14.02.2013 FR 1351253; 14.02.2013 US 201361764636 P
(43) Date de publication de la demande: 23.12.2015
(73) Titulaire: Galderma Research & Development, 06410 Biot (FR)
(72) Inventeur: BOITEAU, Jean-Guy, F-06560 Valbonne (FR); MUSICKI, Branislav, F-06000 Nice (FR)
(74) Mandataire: Cabinet Becker et Associés
(86) Numéro de dépôt international: PCT/FR2014/050314
(87) Numéro de publication internationale: WO 2014/125233

(56) Documents cités:
- WO-A1-2005/121087
- WO-A1-2009/098576
- WO-A1-2010/063774
- WO-A1-2010/081904
- WO-A1-2011/033255
- WO-A1-2011/070080
- WO-A1-2012/122391
- WO-A2-2004/010943
- WO-A2-2007/092681
- PETTERSSON, FREDRIK ET AL: "Synthesis and Evaluation of a Set of 4-Phenylpiperidines and 4-Phenylpiperazines as D2 Receptor Ligands and the Discovery of the Dopaminergic Stabilizer 4-[3-(Methylsulfonyl)phenyl]-1- propylpiperidine (Huntexil, Pridopidine, ACR16)", JOURNAL OF MEDICINAL CHEMISTRY, vol. 53, no. 6, 2010, pages 2510-2520, XP002728055, DOI: 10.1021/JM901689V
- CHIU, GEORGE ET AL: "(Phenylpiperidinyl)cyclohexylsulfonamides : Development of .alpha.1a/1d-selective adrenergic receptor antagonists for the treatment of benign prostatic hyperplasia/lower urinary tract symptoms (BPH/LUTS)", BIOORGANIC & MEDICINAL CHEMISTRY LETTERS, vol. 17, no. 14, 2007, pages 3930-3934, XP002728056, DOI: 10.1016/J.BMCL.2007.04.098

## Description

La présente invention se rapporte à un procédé de synthèse du 4-pipéridin-4-yl-benzène-1,3-diol de formule (I) suivante: 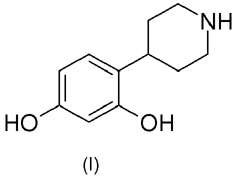 et de ses sels pharmaceutiquement acceptables.

La présente invention concerne également le composé 4-(2,4-dihydroxy-phényl)-4-hydroxy-pipéridine-1-carboxylate de tert-butyle de formule (10)

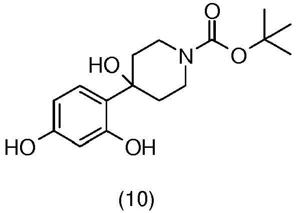

La synthèse de composés analogues à ceux du composé (I) ci-dessus, et répondant à la formule générale suivante : 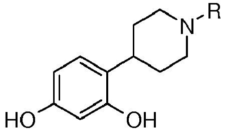 dans laquelle R représente un atome d'hydrogène, ou un radical -COR¹ a été décrite dans la demande de brevet WO 2010/063774. Cette synthèse est réalisée en six étapes (figure 1).

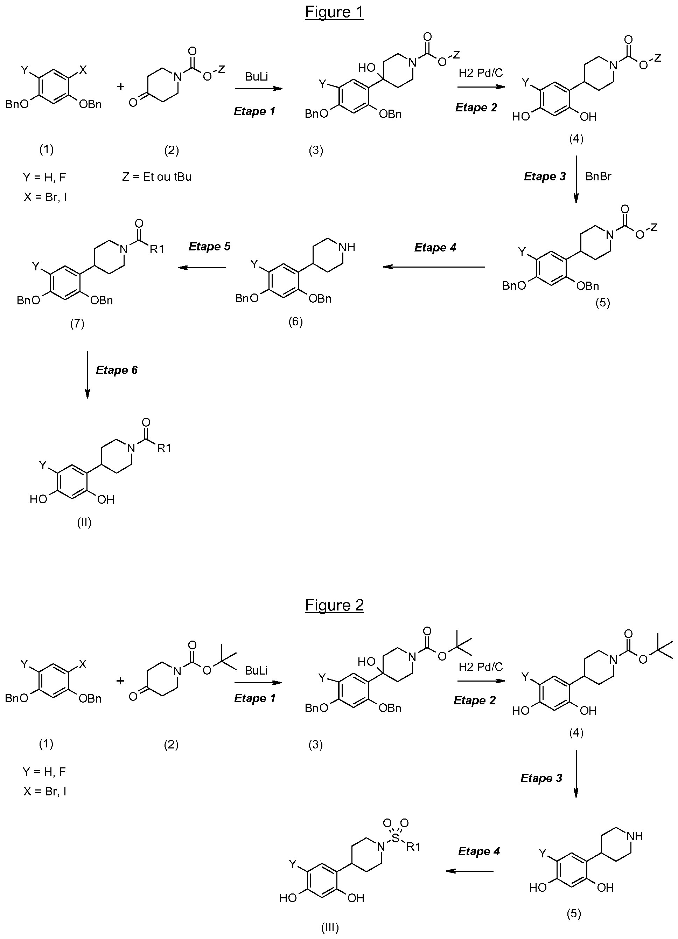

Dans la première étape de ce procédé (figure 1), le 2,4-dibenzyloxy bromobenzène (1) [X=Br ; Y=H], 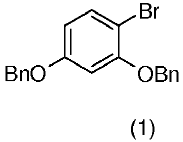 réagit en présence de butyllithium avec des azacycloalkanones de formule générale (2) pour fournir des alcools benzyliques correspondants de formule générale (3)

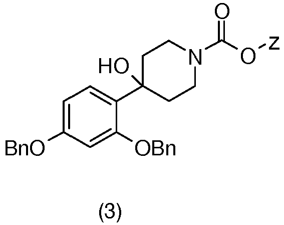

La fonction alcool du composé (3) est ensuite éliminée par une étape de réduction mais ces conditions réductrices provoquent également la déprotection des fonctions phénol (élimination du groupement protecteur benzyle) et donnent le composé (4).

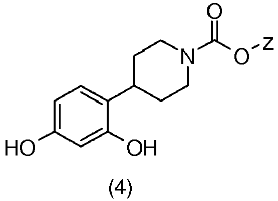

Cette étape de réduction représente un inconvénient dans cette synthèse, car les fonctions phénols du composé 4 doivent à nouveau être protégées dans l'étape suivante pour conduire au composé (5) 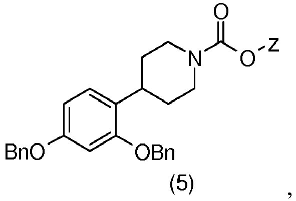 avant de pouvoir éliminer spécifiquement le groupement protecteur carbamate sur l'azote du composé (5) et obtenir le composé (6).

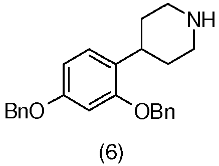

A partir de ce composé (6), un groupement acyle -COR¹ est introduit sur l'azote libre pour conduire au composé (7).

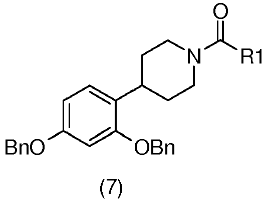

Enfin, une dernière étape de déprotection (débenzylation) des fonctions phénols permet de fournir les composés de formule (II).

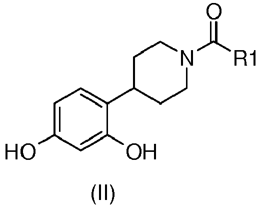

Au total, cette synthèse nécessite deux étapes de déprotection des fonctions phénols (étapes 2 et 6), une étape de déprotection de la fonction azotée (étape 4) et une étape de protection des fonctions phénols (étape 3) pour obtenir les composés de formule (I). Cette succession d'étapes de protection et de déprotection constitue donc un inconvénient majeur de la synthèse telle que décrite dans la demande WO 2010/063774, ledit inconvénient ne permettant pas de transposer cette synthèse à un stade industriel.

La demande de brevet WO 2011/070080 décrit également une synthèse de composés analogues au composé (I) ci-dessus, composés répondant à la formule générale suivante : 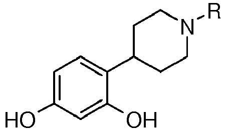 dans laquelle R représente un atome d'hydrogène, ou un radical -SO₂R¹. Cette synthèse est réalisée en quatre étapes (figure 2).

Dans la première étape du procédé tel que décrit dans la demande de brevet WO 2011/070080 (figure 2), le 2,4-dibenzyloxy bromobenzène (1) [X=Br ; Y=H], 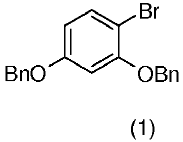 réagit en présence de butyllithium avec des azacycloalkanones de formule générale (2) pour fournir les alcools benzyliques correspondants de formule générale (3)

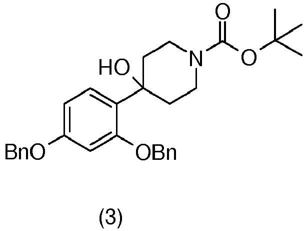

Lors de l'étape suivante, les conditions réductrices utilisées réduisent à la fois, la fonction alcool, et déprotègent les fonctions phénols pour conduire au composé (4).

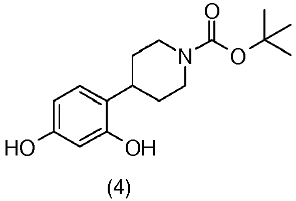

Cette synthèse nécessite encore deux étapes à partir du composé (4) pour fournir les composés de formule (III)

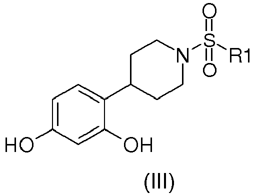

Dans le cadre du développement de composés de formule (II) ou (III), il existe un besoin de disposer d'une méthode simple et économique, permettant de préparer en peu d'étapes, et dans des conditions de sécurité le 4-pipéridin-4-yl-benzène-1,3-diol de formule (I) tout en évitant les inconvénients mentionnés ci-dessus.

La présente invention vise donc à résoudre les problèmes cités ci-dessus en proposant un procédé de synthèse en deux étapes du 4-pipéridin-4-yl-benzène-1,3-diol de formule (I) ou de ses sels, facilement transposable à l'échelle industrielle.

La présente invention concerne donc un procédé de synthèse du 4-pipéridin-4-yl-benzène-1,3-diol tel que défini selon les revendications 1 à 8.

Selon la présente invention, le procédé de synthèse du 4-pipéridin-4-yl-benzène-1,3-diol répondant à la formule générale (I) et de ses sels 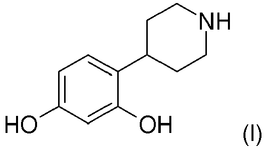 est caractérisé en ce que le composé répondant à la formule générale (10) 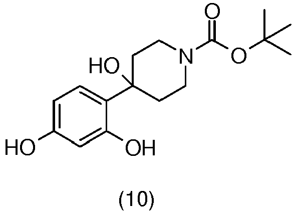 réagit d'abord avec de l'hydrogène en présence d'un catalyseur à base de palladium dans un solvant polaire, et qu'il réagit ensuite avec un acide minéral ou organique.

De préférence, le solvant polaire est choisi dans le groupe comprenant les alcools comme le méthanol, l'éthanol et l'isopropanol par exemple, les acides carboxyliques comme l'acide acétique par exemple, les esters comme l'acétate d'éthyle par exemple, les éthers comme le tétrahydrofurane par exemple, l'eau et un mélange de ces solvants. Avantageusement, le solvant polaire est un alcool choisi parmi le méthanol, l'éthanol et l'isopropanol.

De préférence, le catalyseur à base de palladium est choisi dans le groupe constitué par le palladium sur charbon, l'hydroxyde de palladium et l'acétate de palladium.

De préférence, la pression d'hydrogène utilisée dans le procédé de l'invention est comprise entre 1 et 10 bars.

Selon la présente invention, le composé répondant à la formule générale (10) est obtenu en faisant réagir du résorcinol avec l'azacycloalkone de formule (9) 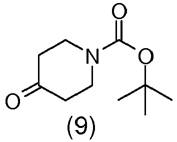 en présence d'une base et dans un solvant polaire.

De préférence, le solvant polaire est choisi dans le groupe comprenant l'eau et les alcools, de préférence le méthanol, l'éthanol, l'isopropanol, le n-butanol et le tert-butanol.

De préférence la base est choisie dans le groupe comprenant l'hydroxyde de sodium, l'hydroxyde de potassium, l'hydroxyde de lithium, les alcoolates de métaux, de préférence le méthanolate de sodium, le tertio-butylate de sodium, le tertio-butylate de potassium ou le tertio-butylate de lithium.

De préférence, le résorcinol et l'azacycloalkanone (9) sont utilisés dans un rapport molaire résorcinol/azacycloalkanone compris entre 1 et 8.

Un autre objet de l'invention concerne le 4-(2,4-dihydroxy-phényl)-4-hydroxy-pipéridine-1-carboxylate de tert-butyle de formule (10)

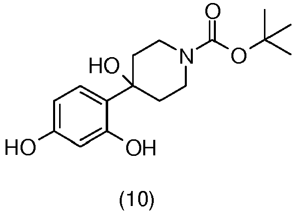

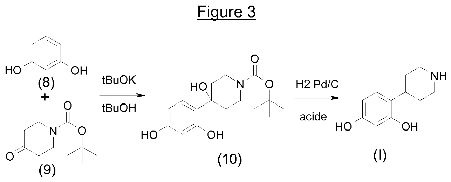

Tel qu'illustré à la figure 3, le nouveau procédé de synthèse du 4-pipéridin-4-yl-benzène-1,3-diol (I), objet de la présente invention, est une synthèse courte comprenant 2 étapes.

Cette nouvelle voie de synthèse utilise le résorcinol (8) comme produit de départ, ce qui, économiquement, est très avantageux comparé au prix du 2,4-dihydroxy bromobenzène (1) utilisé dans les synthèses décrites dans les demandes WO 2010/063774 et WO 2011/070080.

La première étape de cette nouvelle voie de synthèse est une étape originale de couplage du résorcinol (8) avec l'azacycloalkanone (9) en présence d'une base. La base utilisée est choisie de préférence dans le groupe comprenant l'hydroxyde de sodium, l'hydroxyde de potassium, l'hydroxyde de lithium, les alcoolates de métaux comme le méthanolate de sodium, le tertio-butylate de sodium ou encore le tertio-butylate de potassium ou encore le tertio-butylate de lithium par exemple. Cette étape est réalisée dans un solvant polaire, de préférence dans l'eau ou dans des alcools ou encore dans un mélange de ces solvants et permet d'obtenir directement le composé (10). Les alcools sont choisis de préférence dans le groupe comprenant le méthanol, l'éthanol, l'isopropanol, le n-butanol et encore le tert-butanol.

Lors de cette première étape, un excès de résorcinol (8) est de préférence utilisé pour réagir sur l'azacycloalkanone (9). Plus spécifiquement, le rapport molaire résorcinol (8) / azacycloalkanone (9) est compris entre 1 et 8 et, de préférence, entre 2 et 4.

La température à laquelle est effectuée cette étape de couplage représente un autre avantage de cette nouvelle voie de synthèse. En effet, cette première étape est réalisée à température ambiante, dans l'eau ou dans les alcools, ce qui permet d'éviter l'étape cryogénique (-78°C) des procédés divulgués dans WO 2010/063774 et dans WO 2011/070080. D'autre part, l'intermédiaire réactionnel (10) de cette première étape, est obtenu sous forme cristalline et permet de s'affranchir des étapes de purification. Ainsi, les techniques de séparation sur colonne de chromatographie pour isoler l'intermédiaire (10) ne sont pas nécessaires, ce qui constitue un autre avantage pour la transposition de ce procédé à une échelle industrielle.

La seconde étape de cette nouvelle voie de synthèse est une réaction d'hydrogénation de l'intermédiaire (10) pour obtenir le 4-pipéridin-4-yl-benzène-1,3-diol (I).

L'hydrogénation de l'intermédiaire (10) est réalisée dans un solvant polaire. Les solvants polaires préférés sont choisis dans le groupe comprenant les alcools comme le méthanol par exemple, les acides carboxyliques comme l'acide acétique par exemple, les esters comme l'acétate d'éthyle par exemple, les éthers comme le tétrahydrofurane par exemple, l'eau et un mélange de ces solvants. Les alcools sont choisis de préférence dans le groupe comprenant le méthanol, l'éthanol et l'isopropanol. L'hydrogénation est réalisée en présence d'un catalyseur à base de palladium. Les catalyseurs préférés sont choisis dans le groupe constitué par le palladium sur charbon, l'hydroxyde de palladium, l'acétate de palladium, ou encore tout autre catalyseur de réduction connu de l'homme du métier. La pression d'hydrogène utilisée est comprise entre 1 et 10 bars et de préférence entre 3 et 7 bars.

Cette étape présente l'avantage de pouvoir générer le 4-pipeéidin-4-yl-benzene-1,3-diol (I) sous forme de sel après addition d'un acide minéral ou organique dans le milieu réactionnel. Le groupement carbamate de tert-butyle protégeant l'azote du composé (10) est alors clivé *in situ* par l'acide minéral ou organique pour fournir le 4-pipéridin-4-yl-benzène-1,3-diol (I) sous forme de sel.

Les acides minéraux utilisés pour obtenir une forme saline du 4-pipéridin-4-yl-benzene-1,3-diol (I) sont choisis dans le groupe comprenant l'acide chlorhydrique, et l'acide sulfurique par exemple.

Les acides organiques utilisés pour obtenir une forme saline du 4-pipéridin-4-yl-benzene-1,3-diol (I) sont choisis dans le groupe comprenant l'acide trifluorométhane sulfonique, et l'acide trifluoroacétique.

Ce procédé présente donc l'avantage de fournir le 4-pipéridin-4-yl-benzène-1,3-diol (I) ou un de ses sels en un maximum de 2 étapes réactionnelles et est donc beaucoup plus facilement transposable à l'échelle industrielle que les procédés de l'art antérieur.

### Description des Figures

- Figure 1 :: Préparation des composés de formule (II) décrite dans WO 2010/063774
- Figure 2 :: Préparation des composés de formule (III) décrite dans WO 2011/070080
- Figure 3:: Procédé de préparation du composé de l'invention de formule (I) et de ses sels.

### Exemples

Les exemples suivants sont maintenant présentés en vue d'illustrer le procédé tel que décrit précédemment.

### Exemple 1: Chlorhydrate de 4-pipéridin-4-yl-benzène-1,3-diol

### Etape 1 :

### Ester tert-butylique de l'acide 4-(2,4-dihydroxy-phényl)-4-hydroxy-pipéridine-1-carboxylique.

Dans un réacteur de 4 litres équipé d'un agitateur mécanique et d'un thermomètre, on place du résorcinol (371.25 g) dans du tert-butanol (2 L) à température ambiante. A ce mélange hétérogène, on ajoute ensuite du tert-butoxyde de potassium (378,0 g) pendant 5 min. La température s'élève à 55°C et le mélange réactionnel devient noir. La réaction est agitée pendant 30 min permettant au mélange de refroidir à 38°C. Ensuite, on ajoute de la 4-oxo-pipéridine-1-carboxylate de tert-butyle (167,9 g) pendant 2 à 3 minutes dans le mélange réactionnel. L'ajout est endothermique, abaissant la température à 35°C. La réaction est ensuite agitée pendant 1h jusqu'à température ambiante. Le mélange réactionnel est ensuite ajouté à une solution de NaH₂PO₄ (700 g) dans l'eau (5 L) et la phase aqueuse est ensuite extraite avec 4 litres d'un mélange Acétate d'éthyle/heptane (1/1). La phase organique est séparée et lavée avec de l'eau (10 x 5 L). Cette opération est réalisée jusqu'à disparition complète du résorcinol de la phase aqueuse (Contrôle par CCM). La phase organique est ensuite séchée sur Na₂SO₄ et les solvants sont évaporés pour donner 263 g de résidu. Le résidu est dissout dans un minimum de dichlorométhane et est filtré sur un patch de gel silice en utilisant avec d'un mélange Acétate d'éthyle/heptane (1/1). Après évaporation du solvant (concentration), un solide commence à cristalliser lentement. Au bout de 30 minutes, 117 g de 4-(2,4-dihydroxy-phényl)-4-hydroxy-pipéridine-1-carboxylate de tert-butyle sont obtenus. (Rdt = 45%).
**RMN ¹H (DMSO D6, 400 MHz):** 1.40 (s, 9H); 1.52 (d, J = 12.8 Hz, 2 H); 2.10 (m, 1H); 3.08 (sl, 2H); 3.79 (sl, 2H); 5.35 (sl, 1H); 6.17 (dd, *J* =2.4 Hz, 8.3 Hz, 1H); 6.21 (d, *J* = 2.4Hz, 1H); 7.08 (d, *J* = 8.2 Hz, 1H); 9.09 (sl, 1H); 9.47 (sl, 1H).

### Etape 2:

### Chlorhydrate de 4-Pipéridin-4-yl-benzène-1,3-diol

Dans un réacteur d'hydrogénation de 1 L, on dissout 70 g de 4-(2,4-dihydroxy-phényl)-4-hydroxy-pipéridine-1-carboxylate de tert-butyle (0,226 mol) dans 500 ml d'acide acétique glacial avec 4 g de palladium/C (Pd/C) à 10% et le mélange est hydrogéné à 5 bars et à 35°C pendant 3 heures. Au cours de l'hydrogénation, la température s'élève à 65°C. Après 3 h, 500 ml d'acétate éthyle sont ajoutés au mélange réactionnel et la solution est filtrée sur célite. Sur ce filtrat, on ajoute ensuite, goutte à goutte, 150 ml d'une solution 4M HCl dissouts dans l'acétate d'éthyle et, après 2 heures d'agitation, le précipité formé est filtré, permettant d'obtenir 36 g de chlorhydrate de 4-pipéridin-4-yl-benzène-1,3-diol sous forme de cristaux blancs.
Rdt = 69%
**RMN ¹H (DMSO D6, 400 MHz):** 1.80 (m, 4H); 2.92 (m, 3H); 3.28 (d, *J* = 12 Hz, 2H); 6.18 (dd, *J =2.4* Hz, 8.3 Hz, 1 H); 6.34 (d, *J* = 2.4Hz, 1 H); 6.77 (d, *J* = 8.2 Hz, 1 H); 8.93 (sl, 1 H); 9.10 (sl, 1 H); 9.36 (s, 1 H).
**RMN 13C (DMSO D6, 100 MHz) :** 28.3; 32.4; 43.8; 102.5; 106.1; 121.1; 126.5; 155.3; 156.5.

## Revendications

1. Procédé de synthèse du 4-piperidin-4-yl-benzene-1,3-diol répondant à la formule générale (I) et de ses sels 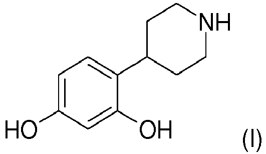 **caractérisé en ce que** le composé répondant à la formule générale (10) 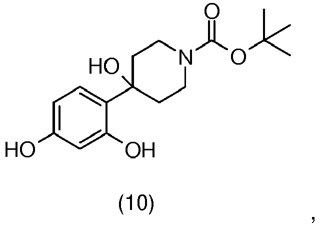 est obtenu en faisant réagir du résorcinol avec l'azacycloalkanone de formule (9) 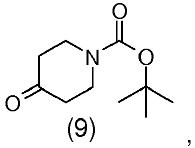 en présence d'une base et dans un solvant polaire, et **en ce que** ledit composé de formule (10) réagit d'abord avec de l'hydrogène en présence d'un catalyseur à base de palladium dans un solvant polaire, et qu'il réagit ensuite avec un acide minéral ou organique.

2. Procédé selon la revendication 1, **caractérisé en ce que** le solvant polaire utilisé lors de la réaction avec l'hydrogène en présence d'un catalyseur à base de palladium est choisi dans le groupe comprenant les alcools, de préférence le méthanol, les acides carboxyliques, de préférence l'acide acétique, les esters, de préférence l'acétate d'éthyle, les éthers de préférence le tetrahydrofuranne, l'eau et un mélange de ces solvants.

3. Procédé selon la revendication 2, **caractérisé en ce que** les alcools sont choisis dans le groupe comprenant le méthanol, l'éthanol et l'isopropanol.

4. Procédé selon la revendication 1, **caractérisé en ce que** le solvant polaire utilisé lors de la réaction du résorcinol avec l'azacycloalkanone de formule (9) est choisi dans le groupe comprenant l'eau et les alcools, de préférence le méthanol, l'éthanol, l'isopropanol, le n-butanol et le tert-butanol.

5. Procédé selon la revendication 1, **caractérisé en ce que** la base est choisie dans le groupe comprenant l'hydroxyde de sodium, l'hydroxyde de potassium, l'hydroxyde de lithium, les alcoolates de métaux, de préférence le méthanolate de sodium, le tertio-butylate de sodium, le tertio-butylate de potassium, ou le tertio-butylate de lithium.

6. Procédé selon la revendication 1, **caractérisé en ce que** le catalyseur à base de palladium est choisi dans le groupe constitué par le palladium sur charbon, l'hydroxyde de palladium et l'acétate de palladium.

7. Procédé selon la revendication 1, **caractérisé en ce que** la pression d'hydrogène utilisée est comprise entre 1 et 10 bars.

8. Procédé selon la revendication 1, **caractérisé en ce que** le résorcinol et l'azacycloalkanone (9) sont utilisés dans un rapport molaire résorcinol/azacycloalkanone compris entre 1 et 8.

9. 4-(2,4-dihydroxy-phényl)-4-hydroxy-pipéridine-1-carboxylate de tert-butyle de formule (10)

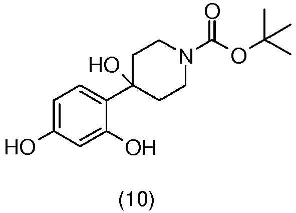

## Patentansprüche

1. Verfahren zur Synthese von 4-Piperidin-4-yl-benzol-1,3-diol, das der allgeneinen Formel (I) entspricht, und Salzen davon 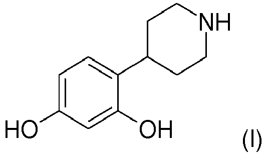 **dadurch gekennzeichnet, dass** die Verbindung, die der allgemeinen Formel (10) entspricht, 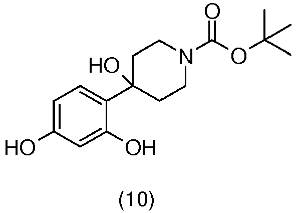 durch Reaktion von Resorcin mit dem Azacycloalkanon der Formel (9) 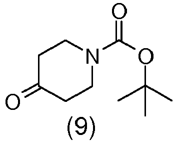 in Gegenwart einer Base und in einem polaren Lösemittel erhalten wird, und **dadurch gekennzeichnet, dass** besagte Verbindung der Formel (10) zuerst mit Wasserstoff in Gegenwart eines Katalysators auf Palladium-Basis in einem polaren Lösemittel umgesetzt wird, und dass sie anschließend mit einer organischen oder anorganischen Säure umgesetzt wird.

2. Verfahren gemäß Anspruch 1, **dadurch gekennzeichnet, dass** das polare Lösemittel, das bei der Reaktion mit Wasserstoff in Gegenwart eines Katalysators auf Palladium-Basis eingesetzt wird, aus der Gruppe ausgewählt wird, umfassend Alkohole, bevorzugt Methanol, Carbonsäuren, bevorzugt Essigsäure, Ester, bevorzugt Essigsäureethylester, Ether, bevorzugt Tetrahydrofuran, Wasser und ein Gemisch dieser Lösemittel.

3. Verfahren gemäß Anspruch 2, **dadurch gekennzeichnet, dass** die Alkohole aus der Gruppe ausgewählt werden, umfassend Methanol, Ethanol und Isopropanol.

4. Verfahren gemäß Anspruch 1, **dadurch gekennzeichnet, dass** das polare Lösemittel, das bei der Reaktion von Resorcin mit dem Azacycloalkanon der Formel (9) eingesetzt wird, aus der Gruppe ausgewählt wird, umfassend Wasser und Alkohole, bevorzugt Methanol, Ethanol, Isopropanol, n-Butanol und tert.-Butanol.

5. Verfahren gemäß Anspruch 1, **dadurch gekennzeichnet, dass** die Base aus der Gruppe ausgewählt wird, umfassend Natriumhydroxid, Kaliumhydroxid, Lithiumhydroxid, Metallalkoholate, bevorzugt Natriummethanolat, Natrium-tert.-butanolat, Kalium-tert.-butanolat oder Lithium-tert.-butanolat.

6. Verfahren gemäß Anspruch 1, **dadurch gekennzeichnet, dass** der Katalysator auf Palladium-Basis aus der Gruppe ausgewählt wird, bestehend aus Palladium auf Kohle, Palladiumhydroxid und Palladiumacetat.

7. Verfahren gemäß Anspruch 1, **dadurch gekennzeichnet, dass** der eingesetzte Wasserstoffdruck zwischen 1 und 10 bar beträgt.

8. Verfahren gemäß Anspruch 1, **dadurch gekennzeichnet, dass** Resorcin und Azacycloalkanon (9) in einem Resorcin/Azacycloalkanon-Molverhältnis zwischen 1 und 8 eingesetzt werden.

9. tert.-Butyl-4-(2,4-dihydroxy-phenyl)-4-hydroxy-piperidin-1-carboxylat der Formel (10)

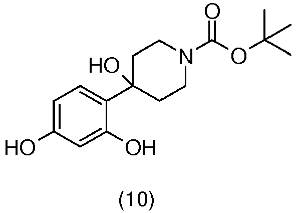

## Claims

1. A method of synthesizing 4-piperidin-4-yl-benzene-1,3-diol corresponding to the general formula (I) and the salts thereof 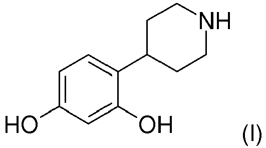 wherein the compound corresponding to the general formula (10) 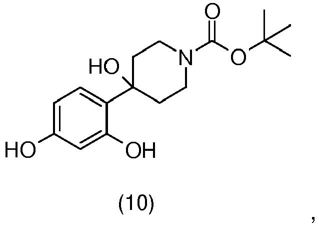 is obtained by reacting the resorcinol with the azacycloalkanone of formula (9) 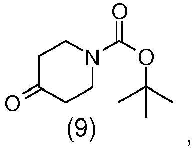 in the presence of a base and in polar solvent, and wherein said compound of formula (10) reacts first with hydrogen in the presence of a palladium-based catalyst in polar solvent, and then reacts with a mineral or organic acid.

2. The method according to claim 1, wherein the polar solvent used in the reaction with hydrogen in the presence of a palladium-based catalyst is selected from the group consisting of alcohols, preferably methanol, carboxylic acids, preferably acetic acid, esters, preferably ethyl acetate, ethers, preferably tetrahydrofuran, water, and a mixture of said solvents thereof.

3. The method according to claim 2, wherein said alcohols are selected from the group consisting of methanol, ethanol, and isopropanol.

4. The method according to claim 1, wherein the polar solvent used in the reaction of resorcinol with the azacycloalkanone of formula (9) is selected from the group consisting of water and alcohols, preferably methanol, ethanol, isopropanol, n-butanol and tert-butanol.

5. The method according to claim 1, wherein the base is selected from the group consisting of sodium hydroxide, potassium hydroxide, lithium hydroxide, metal alcoholates, preferably sodium methanolate, sodium tert-butylate, potassium tert-butylate, or lithium tert-butylate.

6. The method according to claim 1, wherein the palladium-based catalyst is selected from the group consisting of palladium on carbon, palladium hydroxide and palladium acetate.

7. The method according to claim 1, wherein the hydrogen pressure applied is comprised between 1 and 10 bars.

8. The method according to claim 1, wherein the resorcinol and the azacycloalkanone (9) are used in a resorcinol/azacycloalkanone molar ratio comprised between 1 and 8.

9. Tert-butyl 4-(2,4-dihydroxy-peényl)-4-hydroxy-piperidine-1-carboxylate of the formula (10)
